# EUROPEAN PATENT SPECIFICATION

(11) **EP 1 833 423 B1**
(45) Date of publication and mention of the grant of the patent: **16.04.2014**
(21) Application number: 05854545.0
(22) Date of filing: 16.12.2005
(51) Int. Cl.: A61F 2/16

(54) **PRELOADED IOL INJECTOR AND METHOD**
VORBELADENER IOL-INJEKTOR UND VERFAHREN
DISPOSITIF INJECTEUR DE LENTILLE INTRA-OCULAIRE PRECHARGE ET PROCEDE

(30) Priority: 29.12.2004 US 25406
(43) Date of publication of application: 19.09.2007
(73) Proprietor: BAUSCH & LOMB INCORPORATED, Rochester, New York 14604 (US)
(72) Inventor: PYNSON, Joel, F-31400 Toulouse (FR); BESSIERE, Benoit, F-31280 Dremil Lafage (FR); RATHERT, Brian, D., Largo, Florida 33778 (US)
(74) Representative: Vossius & Partner
(86) International application number: PCT/US2005/045857
(87) International publication number: WO 2006/071597

(56) References cited:
- WO-A-2004/052241
- WO-A-2005/030097
- FR-A- 2 848 182
- US-A- 5 873 879
- US-B1- 6 183 513

## Description

### Background of the Invention

The present invention relates to ophthalmic surgical devices and methods. More particularly, the present invention relates to a device for inserting an intraocular lens (IOL) into an eye wherein the IOL may be conveniently preloaded in and packaged together with the injector device.

IOLs are artificial lenses used to replace the natural crystalline lens of the eye when the natural lens has cataracts or is otherwise diseased. IOLs are also sometimes implanted into an eye to correct refractive errors of the eye in which case the natural lens may remain in the eye together with the implanted IOL. The IOL may be placed in either the posterior chamber or anterior chamber of the eye. IOLs come in a variety of configurations and materials. Some common IOL styles include the so-called open-looped haptics which include the three-piece type having an optic and two haptics attached to and extending from the optic; the one-piece type wherein the optic and haptics are integrally formed (e.g., by machining the optic and haptics together from a single block of material); and also the closed looped haptic IOLs. Yet a further style of IOL is called the plate haptic type wherein the haptics are configured as a flat plate extending from opposite sides of the optic. The IOL may be made from a variety of materials or combination of materials such as PMMA, silicone, hydrogels and silicone hydrogels, etc.

Various instruments and methods for implanting the IOL in the eye are known. In one method, the surgeon simply uses surgical forceps having opposing blades which are used to grasp the IOL and insert it through the incision into the eye. While this method is still practiced today, more and more surgeons are using more sophisticated IOL inserter devices which offer advantages such as affording the surgeon more control when inserting the IOL into the eye. IOL inserter devices have recently been developed with reduced diameter insertion tips which allow for a much smaller incision to be made in the cornea than is possible using forceps alone. Smaller incision sizes (e.g., less than about 3mm) are preferred over larger incisions (e.g., about 3.2 to 5+mm) since smaller incisions have been attributed to reduced post-surgical healing time and complications such as induced astigmatism.

Since IOLs are very small and delicate articles of manufacture, great care must be taken in their handling. In order for the IOL to fit through the smaller incisions, they need to be folded and/or compressed prior to entering the eye wherein they will assume their original unfolded/uncompressed shape. The IOL inserter device must therefore be designed in such a way as to permit the easy passage of the IOL through the device and into the eye, yet at the same time not damage the delicate IOL in any way. Should the IOL be damaged during delivery into the eye, the surgeon will most likely need to extract the damaged IOL from the eye and replace it with a new IOL, a highly undesirable surgical outcome.

Thus, as explained above, the IOL inserter device must be designed to permit easy passage of the IOL therethrough. It is equally important that the IOL be expelled from the tip of the IOL inserter device and into the eye in a predictable orientation and manner. Should the IOL be expelled from the tip too quickly or in the wrong orientation, the surgeon must further manipulate the IOL in the eye which could result in trauma to the surrounding tissues of the eye. It is therefore highly desirable to have an inserter device which allows for precise loading of the IOL into the inserter device and which will pass and expel the IOL from the inserter device tip and into the eye in a controlled, predictable and repeatable manner.

To ensure controlled expression of the IOL through the tip of the IOL inserter device, the IOL must first be loaded into the IOL inserter device. The loading of the IOL into the inserter device is therefore a precise and very important step in the process. Incorrect loading of an IOL into the inserter device is oftentimes cited as the reason for a failed IOL delivery sequence. Many IOL injector devices on the market today require the IOL to be loaded into the injector at the time of surgery by the attending nurse and/or surgeon. Due to the delicate nature of the IOL, there is a risk that the nurse and/or surgeon will inadvertently damage the IOL and/or incorrectly load the IOL into the injector device resulting in a failed implantation. Direct handling and/or loading of the IOL into the injector by the nurse and/or surgeon is therefore undesirable.

There remains a need for an IOL inserter and method which removes the need for direct handling of the IOL by the nurse and/or surgeon and which generally simplifies operation of the IOL injector device and IOL delivery process.

### Summary of the Invention

In a broad aspect of the invention, an injector device is provided having a proximal tubular body section having a longitudinal passageway extending between opposite, open ends thereof, a shuttle which holds the IOL in an initially unstressed state, and a nozzle section which are packaged and then assembled together at the time of surgery. A plunger component is inserted into the proximal open end of the tubular body and telescopes within the longitudinal passageway thereof. The IOL is preloaded in an unstressed condition in the shuttle component of the device (i.e., in a condition where at least the IOL optic is not compressed or folded). The shuttle and IOL are packaged either dry or submersed in a container of sterile solution which maintains the IOL in a hydrated state during shipping and storage, a necessary requirement for IOLs made of certain materials such as acrylic.

In a particularly advantageous embodiment of the invention, the shuttle component is releasably attached to a cover of the storage container whereby the cover may be manually grasped and used to plug the shuttle component (with IOL already contained therein) into the proximal body section of the injector device. At the time of surgery, the nurse or surgeon simply opens the container containing the shuttle and IOL and removes them from the container by grasping the container top to which the shuttle is attached. While still grasping the cover, the shuttle is then plugged into the device body at which time the cover may be detached from the shuttle. A quick release mechanism may be employed to allow for the quick and easy detachment of the cover from the shuttle once the shuttle has been plugged into the device body.

The nozzle section includes a distal tip through which the IOL is ultimately expelled from the injector device and is attached to the device body to complete the assembly. The body, shuttle and nozzle each include a longitudinal passageway which preferably lie along a common longitudinal axis when the body, shuttle and nozzle sections are assembled together. A plunger is provided which telescopes within the device body and may be advanced therein to enter the shuttle component and engage and push the IOL through the shuttle and nozzle, the IOL ultimately being expelled from the injector device at the nozzle tip. The injector device includes means for compressing or otherwise urging the IOL into a smaller cross-section for delivery through the injector. In a preferred embodiment of the invention, the shuttle and nozzle passageways are configured with a narrowing taper towards the distal tip. The plunger is advanced at the proximal end of the injector device causing the distal tip of the plunger to engage the IOL optic. As the plunger is advanced further, the IOL is pushed through the narrowing passageway, thereby compressing the IOL into a smaller cross-section and finally exiting at the nozzle tip and expressed into the eye in the intended manner.

In an alternate embodiment, the shuttle is first attached to the nozzle section and the nozzle/shuttle unit is then attached to the device body.

In yet a further alternate embodiment, the nozzle section and shuttle are connected together and placed in the storage container. In this alternate embodiment, the nozzle and shuttle are releasably connected to the cover and handled as a unit whereby the cover may be manually grasped to plug the nozzle and shuttle unit into the device body.

### Brief Description of the Drawings

Figure 1 is a perspective view of the fully assembled injector device showing the an IOL expressed from the distal tip thereof;
Figure 2a is a perspective view of the shuttle component and IOL packaged in a vial with an outer cover thereof being removed;
Figure 2b is the view of Figure 2a showing the inside cover and shuttle component being removed from the vial;
Figure 3a is a side elevational view showing a user plugging the shuttle component into the proximal body section of the device;
Figure 3b is the view of Fig. 3a showing the shuttle component fully inserted into the proximal body section and the nozzle section being attached to the proximal body section;
Figure 4a is a side elevational view of an alternate embodiment showing the shuttle being connected to the nozzle section;
Figure 4b is a perspective view showing the nozzle section and shuttle combination being connected to the proximal body section;
Figure 4c is a perspective view showing an alternate embodiment where the nozzle section and shuttle are attached together and releasably attached to the vial cover;
Figure 5 is an enlarged, perspective view of the plunger component of the device;
Figure 6 is an enlarged, perspective view of the proximal body section of the device;
Figure 7a is an enlarged, perspective view of the shuttle component of the device;
Figure 7b is the view of Figure 7a showing the shuttle component in the open position and an IOL placed therein; and

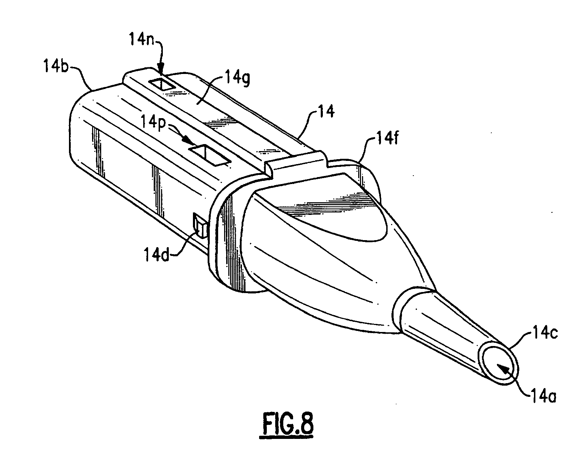

Figure 8 is a perspective view of the nozzle section of the device.

### Detailed Description

The invention comprises a preloaded injector device for injecting an IOL into an eye. The term "preloaded" as used herein means that a packaged component of the injector device includes an IOL positioned therein. Direct handling and loading of an IOL into the injector device is therefore not necessary.

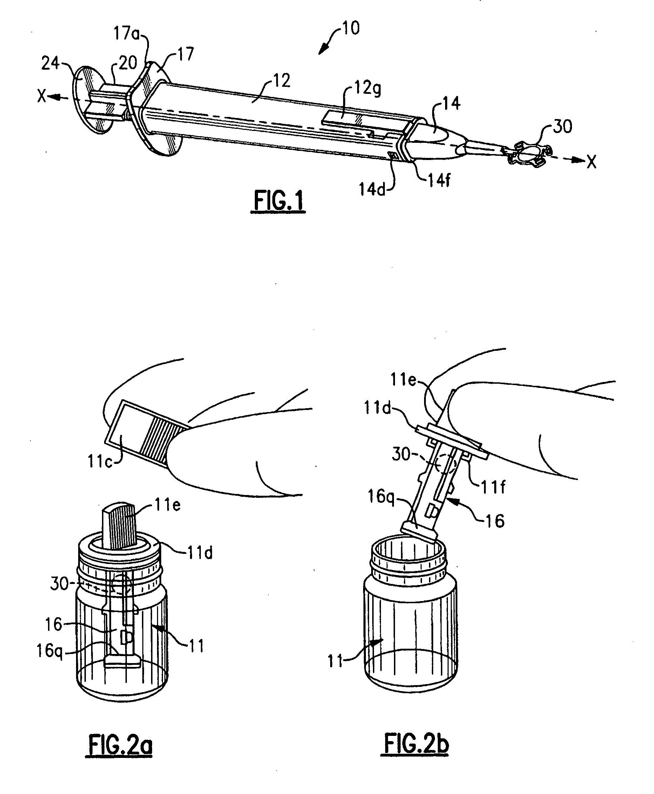

The basic components of injector device 10 include a proximal body section 12, a plunger 20, a distal nozzle section 14, and a shuttle component 16 which are assembled at the time of surgery to ready the device 10 for delivery of an IOL 30 therethrough. An IOL 30 is preloaded into the shuttle component 16 of the device which is packaged in either a dry state or in a hydrated state in a vial 11 (Figs. 2a,b) containing solution to maintain the IOL in a hydrated state during shipping and storage. Whether the IOL is packaged and stored in the dry or wet state depends on the type of material from which the IOL is made. Examples of IOL materials which may be packaged in the dry state include silicone while IOL materials which require wet storage include acrylic.

The different embodiments of the invention will now be briefly described followed by a more detailed description of the individual components thereof.

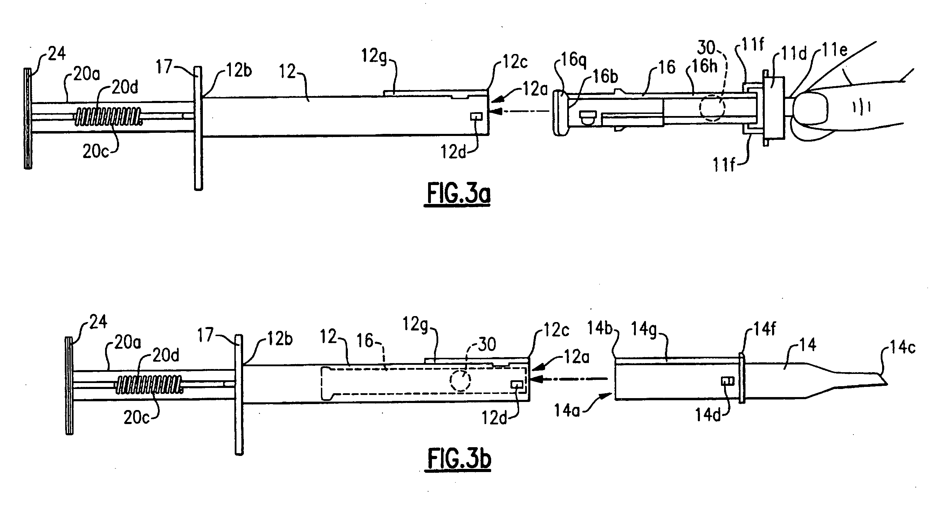

In a first embodiment seen in Figs. 2 and 3, at the time of surgery, the vial 11 is opened and the shuttle 16 having an IOL 30 preloaded therein is removed from the vial. The vial may contain a quantity of storage solution (not shown) if required to maintain the IOL 30 in a hydrated state during storage. Once removed from vial 11, shuttle 16 is plugged into the proximal body section 12 followed by attachment of the distal nozzle section 14 whereupon the device is ready for injecting the IOL 30 into a patient's eye. In an advantageous embodiment, the shuttle is releasably attached to a cover of the vial which may be manually grasped and used to plug the shuttle into the device body at which point the cover is removed from the shuttle.

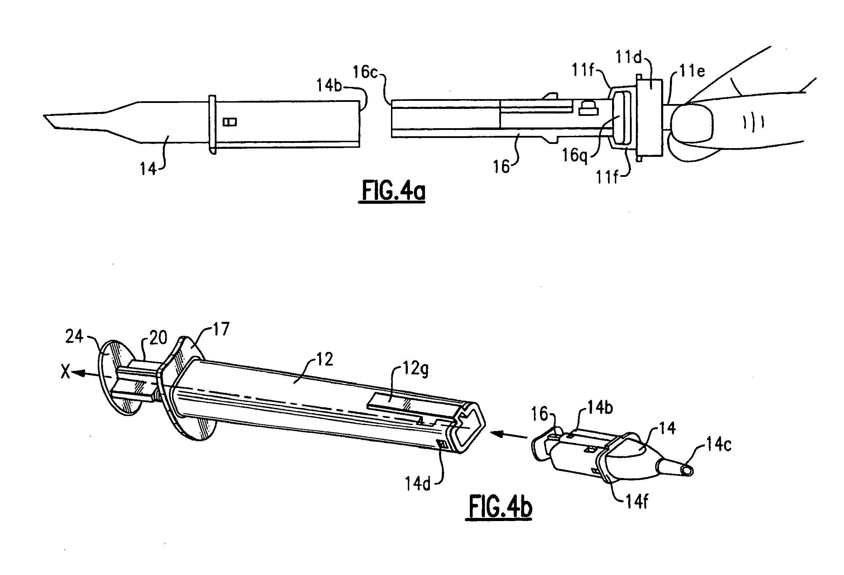

In a second embodiment shown in Figs. 4a,b, at the time of surgery, the vial 11 is opened and the shuttle 16 having IOL 30 preloaded therein is removed from the vial. The shuttle is then plugged into the nozzle section 14 followed by attachment of the nozzle section and shuttle to the proximal body section 12 whereupon the device is ready for injecting an IOL 30 into a patient's eye. In an advantageous embodiment, the shuttle is releasably attached to a cover of the vial which may be manually grasped and used to plug the shuttle into the nozzle section at which point the cover is removed from the shuttle.

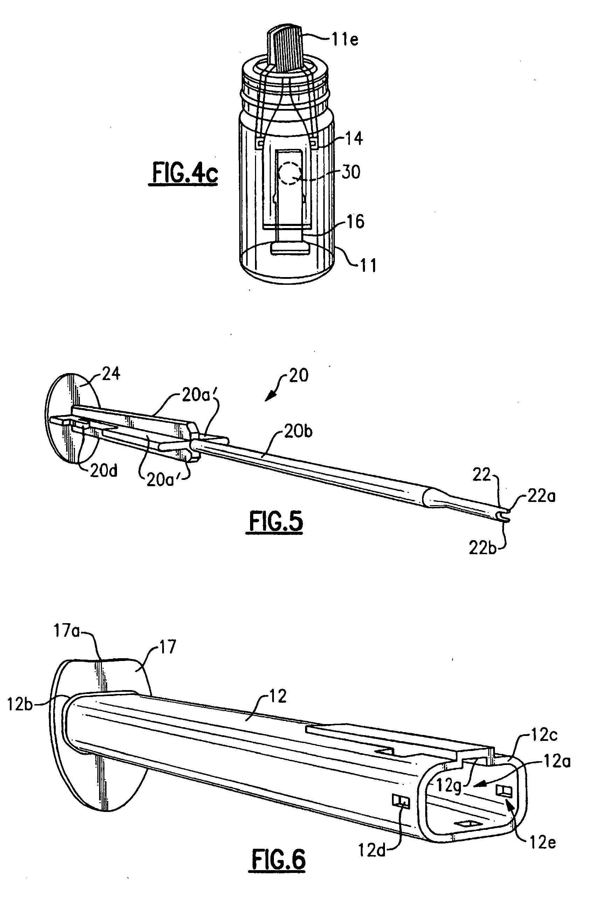

In a third embodiment seen in Fig. 4c, the nozzle section 14 is attached to the shuttle 16 having the IOL preloaded therein and the nozzle/shuttle/IOL combination is releasably connected to the vial cover and sealed in the vial 11. At the time of surgery, the vial 11 is opened and the nozzle/shuttle/IOL combination is removed therefrom and then attached to the proximal body section 12 whereupon the cover is released from the nozzle section. The device is then ready for injecting IOL 30 into a patient's eye.

The proximal body section 12 includes a longitudinal passageway 12a extending between the open proximal and distal ends 12b,12c thereof, respectively. The passageway 12a may assume any desired cross-sectional shape such as a rounded rectangular shape as shown.

The distal nozzle section 14 includes a longitudinal passageway 14a extending between the open proximal end 14b and open distal tip 14c thereof (Fig. 3b). The passageway 14a tapers inwardly toward distal nozzle tip 14c so that the IOL is gradually compressed to a very small cross-section (e.g., sub 3mm) as it exits the device at tip 15c. Once delivered into the patient's eye, the IOL returns to its original shape due to the elastic memory of the material from which it is made.

Shuttle 16 also includes a longitudinal passageway 16a extending between the open proximal end 16b and open distal end 16c thereof. When shuttle 16 is positioned in distal section 14, it is preferred, though not necessary, that the longitudinal passageways 16a, 14a of each are aligned along the same axis X-X. When the proximal body section 12 is attached to the distal nozzle section 14, the longitudinal passageway 12a is aligned along the common axis X-X of the nozzle and shuttle passageways 14a, 16a (Fig. 1).

Referring again to proximal body section 12, a finger flange 17 may be formed at the proximal end 12b thereof for ease in operating the injector device in the manner of a syringe. Finger flange is preferably configured with a straight edge 17a as shown (Fig. 1) for resting device 10 on a flat surface.

A plunger 20 having proximal and distal lengths 20a, 20b, respectively, a distal plunger tip 22, and a thumb press 24 telescopes within the proximal body section 12. When the proximal body section 12, shuttle 16 and nozzle 14 are attached together, the plunger 20 extends sequentially through proximal body section passageway 12a and the shuttle passageway 16a so as to engage and push the IOL 30 through passageway 16a and out distal tip 15c. The IOL delivery sequence will be explained in more detail below.

It is understood that the overall configuration of the injector body 12 may vary from that shown and described herein. It is furthermore understood that the components of the injector device may be made of any suitable material (e.g., polypropylene) and may be wholly or partly opaque, transparent or translucent to better visualize the IOL within the injector device and the IOL delivery sequence. In a preferred embodiment of the injector device, the components thereof which require wet storage in vial 11 are steam sterilized, requiring that the components are made from a material which can withstand the heat generated during steam sterilization. Examples of such materials include, but are not limited to, polypropylene, polycarbonate, polysulfone, ALTEM (by Dupont), and PFA.

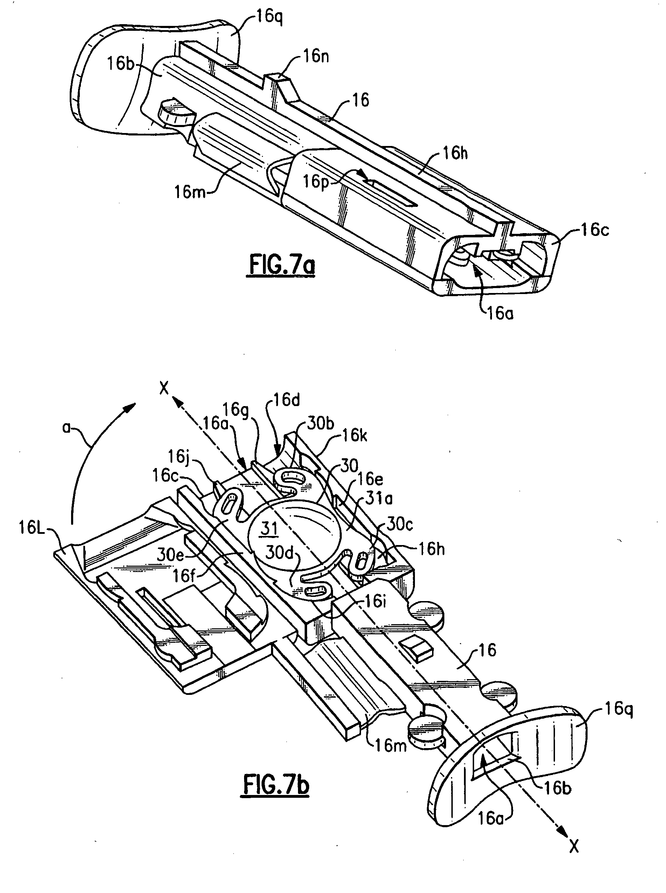

As stated above, shuttle 16 is used for holding an IOL 30 in the preloaded position. As seen best in Figures 7a and 7b, shuttle 16 includes an IOL loading area 16d wherein the IOL 30 is positioned in an unstressed state. Loading area 16d is in open communication with longitudinal passageway 16a and is configured to position the IOL 30 along axis X-X in an unstressed state and may include one or more optic support elements 16e,f each having a radius or other feature for aligning the IOL optic 31 along passageway 16a (and hence also axis X-X) about the periphery 31a thereof. Alternatively or in addition to the optic support elements, one or more haptic support elements 16g-j are provided on shuttle 16, each of which include a radius or other feature for aligning one or more haptics 30b-e which attach to and extend from the optic 31. In this regard, it is understood that the IOL configuration 30 shown and described herein is for discussion purposes only, and that the present invention is not to be limited thereby. The invention may be easily adapted to IOLs of any configuration and type (e.g., IOLs with plate, open or closed loop haptics, anterior chamber IOLs, posterior chamber IOLs, accommodating IOLs (including single and double lens types), etc.). The overall configuration of the IOL shuttle 16 and IOL loading area 16a may thus likewise vary so as to be cooperatively configured with and align the particular IOL style being used with the device. For ease of description, the shuttle embodiment will be described with reference to IOL 30. In all embodiments, the shuttle 16 holds at least the IOL optic 31 in the unstressed state. It is furthermore preferable that shuttle 16 hold the IOL haptics at the correct vault angle (i.e., the angle from which they normally extend from the IOL optic periphery). It is even furthermore preferable that, in the case of an IOL having open looped haptics, the haptic support elements maintain the looped haptics at the correct angle of curvature by constraining the haptics along the outer curved edges thereof. This ensures that the haptic curvature, which is designed and set at manufacture of the haptics, does not increase or bend out of specification during storage of the IOL and shuttle.

At manufacture, the IOL 30 is placed in the shuttle 16. Positioning the IOL 30 in the shuttle 16 may be done by a worker using a pair of forceps, for example, although other methods may be used as desired, including automated or semi-automated means in an assembly line. To facilitate loading of the IOL in the shuttle, the IOL loading area 16a may be formed with two wall sections 16k and 16L which are pivotally connected (e.g., via a living hinge 16m) to enable opening and closing of the IOL loading area 16d. Wall sections 16k and 16L are spread open in a coplanar relationship in the open position of the shuttle loading area 16d. In this open position, IOL loading area 16d is easily accessible and an IOL 30 may be simply placed upon one of the two sections, preferably upon section 16k. This may be done by aligning the IOL optic 31 with the IOL supporting elements 16g,j and aligning the haptics 30b-e with the haptic support elements 16d, 16e, respectively.

Once the IOL 30 is properly positioned in the shuttle IOL loading area 16a, the two sections 16g, 16h are pivoted together (in the direction of arrow "a" in Fig. 7b) to the closed position which encases IOL 30 between the now facing wall sections 16k, 16L (Fig. 7a). With the IOL 30 thus positioned in the shuttle 16, the shuttle 16 is closed and is then deposited into a dry container (for IOL material that may be dry packaged) or a vial 11 with a quantity of storage solution (for IOL material that requires wet storage). For wet packaging, to ensure storage solution reaches the IOL 30, the shuttle may include one or more through-holes 14p, 16p which permit fluid communication with the IOL 30. The container or vial 11 is sealed and sterilized using known methods.

To assist in attaching the shuttle to the distal section in the correct manner, a longitudinal groove 14h (Fig. 6d) may be formed on an inner wall surface of distal section 14 which aligns with a longitudinal flange 16h formed on an outer wall surface of shuttle 16 (Fig. 5b). As such, the shuttle 16 may be slidingly received within distal section 14 with groove 14h and flange 16h providing a "key" to prevent incorrect coupling between the shuttle and distal section. Furthermore, the shuttle 16 and distal section 14 may be fixed in the assembled condition through suitable mechanical locking features. For example, the shuttle 16 may be provided with a detent 16n and the distal section provided with a slot 14n which engage upon full advancement of the shuttle within the distal section. It will thus be realized that the shuttle 16 is then fixed to the distal section 12. It is further noted that the shuttle 16 may be provided with a proximal flange 16q at proximal end 16b to assist in maintaining proper alignment between the proximal section passageway 12a and the shuttle 16. Flange 16q may or may not touch the inner wall surface defining proximal section passageway 12a.

Turning attention to Figs. 2a,b, at the time of surgery, the container or vial 11 is removed from any outer packaging in a sterile field and the outer vial cover 11c is removed to open vial 11 and access shuttle 16. As stated above, shuttle 16 may be releasably attached to the cover 11c, or an inner cap 11d located beneath cover 11c, whereby the user may manually grasp inner cap 11d by a cover flange 11e and lift the shuttle 16 from vial 11 without having to directly handle the shuttle 16.

In the first embodiment described above, while still grasping cap 11d, the user then proceeds to plug the shuttle 16 into the device body 12 as seen in Fig. 3a. Shuttle 16 slides within the longitudinal opening 12a at the distal end 12c of proximal body section 12. Once fully received therein, the user releases cap 11d from shuttle 16. In this regard, it is noted that any appropriate mechanism may be employed to releasably connect shuttle 16 to cap 11d. In the embodiment shown in the figures, one or more latches 11f extend from cap 11d to releasable engage shuttle 16. The latches 11f may be operable between an engaged position and a release position with respect to the shuttle 16, for example, by spring loading the latches biased in the engage position. To move the latches to the release position, the user would squeeze the cap to spread the latches away from each other and thereby release the shuttle. Other shuttle engage and release mechanisms are of course possible and within the scope of the invention.

Once the shuttle 16 is received in the body section 12, nozzle section 14 is attached thereto as seen in Fig. 3b. The nozzle section 14 may include one or more detents 14d for aligning and engaging one or more respective holes 12d formed in body section 12 to interconnect these components. Other connection means are of course possible and within the scope of the invention. Once connected, the device 10 is ready for injecting IOL 30 into an eye.

Referring now to Figs. 4a,b and to the second, alternate embodiment described above, while still grasping cap 11d, the user removes the shuttle 16 from vial 11 as described with reference to the first embodiment. The user then proceeds to plug the shuttle 16 into the nozzle section 14 as seen in Fig. 4a. Shuttle 16 slides within the longitudinal opening 14a at the proximal end 14b of nozzle section 14. Once fully received therein, the user proceeds to connect the nozzle section 12 and shuttle 16 as a unit to the proximal body section 14 as seen in fig. 4b. The user may then release cover 11d from nozzle section 14.

In the third embodiment seen in Fig. 4c, the nozzle section 14 and shuttle 16 (with IOL 30 preloaded therein) are connected together and releasably attached to vial cover 11d for storage in vial 11. The user removes the nozzle section and shuttle together from the vial and attaches the nozzle section 14 to the proximal body section 12 as seen in Fig. 4b whereupon the vial cover 11d may be removed.

Upon further pressing of proximal section 12 against nozzle section 14 results in the two sections attaching together. Various mechanical connection features may be employed to permit the quick and easy attachment of the proximal body section 12 to the nozzle section 14 by simply pressing the two sections together as described above. Such features may include cooperating detents and recesses or a friction fit between the two sections, for example. In the embodiment shown in the Figures, a pair of detents 14d,e (Figs. 6a-d) are provided on the outer wall surface of distal section 14 which align with and engage a pair of through-holes 12d,e formed on proximal section 12 adjacent open distal end 12c thereof (Figs. 3a,b). When the proximal section 12 is pressed against the distal section 14, the detents 14d,e engage the through-holes 12d,e, respectively, and the sections become attached together. A radial flange 14f may be provided on distal section 14 to act as a stop against further advancement of the proximal section 12 on the distal section 14, i.e., to prevent advancement beyond the point of detent engagement. The assembly of the injector device is now complete and the surgeon may proceed to inject the IOL 30 into a patient's eye by inserting tip 14c into an incision formed in the eye and pressing plunger 20 to advance the IOL 30 through and out the nozzle tip 14c (see Fig. 2; the eye not shown for sake of clarity).

Referring to Figures 1, 3a,b and 5, it is seen that the plunger 20 includes distal and proximal plunger shaft lengths 20a, 20b, respectively, having a plunger tip 22 at the distal end thereof and a thumb press 24 at the proximal end thereof for manually operating the injector device. The plunger tip 22 is configured for engaging the IOL optic 31 at the periphery 31a thereof as the plunger 20 is advanced toward the distal tip 14c of distal section 14. It is very important that the plunger tip 22 not damage the IOL optic 31. The plunger tip 22 is thus designed to prevent damage to the IOL optic 31. In the preferred embodiment, the tip is bifurcated into first and second tip portions 22a and 22b, whereby the IOL optic periphery 31a becomes engaged between tip portions 22a, 22b as seen in Figure 2B. It is understood that other plunger tip designs may be used with the present invention as desired. It is furthermore preferred that the plunger shaft is rotationally fixed within passageway 12a to prevent unexpected rotation of the shaft (and thus the tip 22) therein. For example, the plunger shaft may be rotationally fixed by forming the proximal shaft length 20b and passageway 12a non-circular in cross-section as shown.

In a particularly advantageous embodiment, the proximal length 20b of the plunger shaft is provided with one or more elongated flanges 20a' which align with a like number of slots 12a' formed between radially extending fins 21a-d formed on the inner wall surfaces of proximal section 12 adjacent proximal end 12b thereof (Fig. 3c). The purpose of flanges 20a' and slots 12a' is to provide tactile resistance therebetween and thereby allowing the surgeon more precise control and feel when advancing the plunger. The fins 21a-d may be made flexible yet resilient to provide the amount of tactile resistance desired. It is understood that other ways of providing tactile resistance between the plunger and injector body are within the scope of this invention. This provides the surgeon with continuous tactile feedback allowing the surgeon to advance the plunger (and thus the IOL) through the injector device in a very concise and controlled manner. Additionally, the flanges 20a' and slots 12a' help provide proper centering of the plunger shaft 20 and tip 22 relative to axis X-X along which the passageways of the components lie as explained above. Upon full advancement of the plunger, it is desirable to have the plunger automatically retract to some degree upon release of finger pressure against plunger finger press 24. In this regard, a spring 20c may be provided on a finger 20d on shaft length 20a. As the plunger is advanced, the spring 20c will interact with the one or more of the fins 21a-d as the plunger 20 is advanced therethrough.

When it is time to use the injector device, the surgeon selects a package or vial 11 having the appropriate IOL style and power preloaded in the shuttle stored in the vial as described above. The outer packaging is removed in a sterile field of the surgical suite. The proximal section having the plunger coupled thereto is also removed from its associated packaging in the sterile filed. If desired, all the injector components including the vial may be placed in a single outer packaging to present all the injector device components together. The nurse or surgeon proceeds to remove the shuttle and IOL from the vial in the manner described above. While holding the vial cover, the shuttle and IOL are connected to the nozzle section which is then attached to the proximal body section as described above. Once the device 10 is fully assembled as shown in Fig. 1, the surgeon inserts the distal tip 14c into an incision cut into the eye and begins advancing the plunger 20. As the plunger 20 is advanced, the plunger tip 22 engages the optic periphery 31a and pushes IOL 30 forwardly. Upon continued advancement of the plunger 20, the IOL 30 is pushed through the shuttle passageway 16a and is expressed from distal tip 14c and into the eye. As stated above, the spring 20c provides increasing bias in the reverse direction as the plunger reaches the fully advanced position. This occurs as spring 20c is compressed against one or more of the fins 21 a-d. This assists the surgeon in maintaining precise control over plunger (and hence IOL) advancement and allows automatic retraction of the plunger upon relieving the pushing pressure being exerted against the plunger thumb press 24. This is useful for easily executing a second stroke of the plunger in order to engage and manipulate the trailing haptic into place in the eye. This feature, together with the bifurcated plunger tip 22, allows a more precise control and manipulation of the IOL with the plunger tip in-situ than would be possible with an injector device not having these features.

As discussed above, the device may be used for IOLs of any type and style. The configuration of the various component parts may likewise vary to accommodate the particular IOL style being employed with the device. It may thus be realized that the present invention provides an injector device method and apparatus that may be provided in a variety of embodiments.

## Claims

1. A method of packaging an intraocular lens (30) in a portion ofan intraocular lens injection device (10) comprising a shuttle (16), the method comprising the steps of:
a) providing the shuttle (16) and positioning the intraocular lens (30) therein;
b) providing a vial (11) with a cover (11c,11d);
c) releasably connecting said shuttle (16) to said cover (11c,11d) and sealing said shuttle (16) with said intraocular lens (30) therein in said vial (1a).

2. The method of claim 1, and further comprising the steps of:
a) providing a proximal body section (12) of the intraocular lens injection device (10) and a plunger (20) slidably received in said proximal body section (12);
b) opening the vial (11) and removing said cover (11c,11d) and shuttle (16) together from said vial;
c) while holding said cover (11c,11d), attaching the shuttle (16) to said proximal body section (12);
d) removing said cover (11c,11d) from said shuttle(16); and
e) attaching a distal nozzle section (14) of the intraocular lens injection device (10) to said proximal body section (12).

3. The method of claim 1, and further comprising the steps of:
a) providing a proximal body section (12) of the intraocular lens injection device (10) and a plunger (20) slidably received in said proximal body section (12);
b) opening the vial (11) and removing said cover (11c,11d) and shuttle (16) together from said vial (11);
c) while holding said cover (11c, 11d), attaching the shuttle (16) to a distal nozzle section (14) of the intraocular lens injection device (10);
d) removing said cover (11c, 11d) from said shuttle (16); and
e) attaching said distal nozzle section (14) with said shuttle (16) to said proximal body section (12).

4. A method of packaging an intraocular lens (30) in a portion of an intraocular lens injection device (10) comprising the steps of:
a) providing a distal nozzle section (14) of the intraocular lens injection device (10);
b) providing a shuttle (16) and positioning the intraocular lens (30) therein and attaching said shuttle (16) to said distal nozzle section (14);
c) providing a vial (11) with a cover (11c,11d);
d) releasably connecting said distal nozzle section (14) to said cover (11c, 11d) and sealing said distal nozzle section (14), said shuttle (16) and said intraocular lens (30) therein in said vial (11).

5. The method of claim 4, and further comprising the steps of:
a) providing a proximal body section (12) of the intraocular lens injection device (10) and a plunger (20) slidably received in said proximal body section (12);
b) opening the vial (11) and removing said cover (11c,11d) said distal nozzle section (14) and said shuttle (16) together from said vial (11);
c) while holding said cover (11c,11d), attaching said distal nozzle section (14) to said proximal body section (12); and
d) removing said cover (11c,11d) from said distal nozzle section (14).

6. The method of claim 2, 3 or 5 further comprising the step of packaging said proximal body section (12) and said distal nozzle section (14) separately from said vial (11).

7. The method of claim 1 further comprising the step of sterilizing said shuttle (16) and said intraocular lens (30) when sealed in said vial (11).

8. The method of claim 7 wherein said sterilization step is steam sterilization.

9. The method of claim 1 wherein said shuttle is snap fit to said proximal body section (12).

10. The method of claim 1 wherein said proximal body section (12) and said distal nozzle section (14) are snap fit together,

11. A method of preparing an injector device for use, comprising the steps. of:
a) providing a proximal body section (12) of the injector device having a longitudinal passageway;
b) providing a distal nozzle section (14) of the injector device having a longitudinal passageway;
c) providing a shuttle (16) having a longitudinal passageway and an intraocular lens (30) positioned in said shuttle (16);
d) providing a vial (11) having a cover (11c,11d) for sealing an open end of said vial (11), the cover (11c,11d) being releasably connected to said shuttle (16), said cover (11c,11d) positioned to seal said shuttle (16) and intraocular lens (30) inside said vial (11);
e) removing said shuttle (16) from said vial (11) using said cover (11c, 11d);
f) attaching said shuttle (16) to said proximal body section (12) or said distal nozzle section (14) using said cover (11c, 11d); and
g) releasing said vial cover (11c, 11d) from said shuttle (16).

12. The method of claim 11, further comprising attaching said distal nozzle section (14) to said proximal body section (12).

13. A kit for preparing an injector device (10) for use, comprising:
a) a proximal body section (12) of the injector device (10) having a longitudinal passageway;
b) a distal nozzle section (14) of the injector device (10) having a longitudinal passageway;
c) a shuttle (16) having a longitudinal passageway and an intraocular lens (30) positioned in said shuttle (16);
d) a vial (11) having a cover (11c, 11d) for sealing an open end of said vial (11), the cover (11c, 11d) being releasably connected to said shuttle (16), said cover (11c, 11d) positioned to seal said shuttle (16) and intraocular lens (30) inside said vial (11); wherein
e) said shuttle (16) is removable from said vial (11) using said cover (11c, 11d);
f) said shuttle (16) is attachable to said proximal body section (12) or said distal nozzle section (14) using said cover (11c, 11d); and
g) said vial cover (11c ,11d) is releasable from said shuttle (16).

14. The kit of claim 13, further comprising means for attaching said nozzle section (14) to said proximal body section (12).

15. The kit of claim 13 or 14 wherein said proximal body section (12) and said distal nozzle section (14) are coaxially attached and said distal nozzle section (14) includes a distal tip at the end thereof opposite said proximal body section (12) wherethrough said intraocular lens (30) may be expressed further comprising:
a plunger (30) in telescoping relation within said proximal body (12) and distal nozzle sections (14); and wherein
said plunger (20) is adapted to be entirely advanced through said distal nozzle section (14) thereby expressing said intraocular lens (30) from said distal tip.

16. The kit of any of claims 13 to 15 wherein said shuttle (16) is adapted to be snap fit to said proximal body section (12).

17. The kit of any of claims 13 to 16 wherein said proximal body section (12) and said distal nozzle section (14) are adapted to be snap fit together.

18. A vial (11) having a cover (11c ,11d) for sealing an open end of said vial (11) and a shuttle (16) having a longitudinal passageway and an intraocular lens (30) positioned in said shuttle (16),
said cover (11c ,11d) positioned to seal said shuttle (16) and intraocular lens (30) inside said vial (11); wherein said shuttte (16) is removable from said vial (11) using said cover (11c ,11d),
wherein said shuttle (16) is attachable to a proximal body section (12) or a distal nozzle section (14) of an injector device (10) using said cover (11c ,11d).

19. The vial of claim 18 wherein said shuttle (16) is adapted to be snap fit to said proximal body section (12).

20. The vial of any of claims 18 and 19 wherein said vial cover (11c ,11d) is releasable from said shuttle (16).

## Patentansprüche

1. Verfahren zum Laden einer Intraokularlinse (30) in einen Abschnitt einer Intraokularlinse-Injektionsvorrichtung (10) mit einem Shuttle (16), wobei das Verfahren die folgenden Schritte aufweist:
a) Bereitstellen des Shuttles (16) und Anordnen der Intraokularlinse (30) darin;
b) Bereitstellen eines Behälters (11) mit einem Deckel (11c,11d);
c) lösbares Verbinden des Shuttles (16) mit dem Deckel (11c,11d) und Versiegeln des Shuttles (16) mit der darin angeordneten Intraokularlinse (30) in dem Behälter (11).

2. Verfahren nach Anspruch 1, ferner mit den folgenden Schritten:
a) Bereitstellen eines proximalen Körperabschnitts (12) der Intraokularlinse-Injektionsvorrichtung (10) und eines gleitbar in dem proximalen Körperabschnitt (12) untergebrachten Kolbens (20);
b) Öffnen des Behälters (11) und Abnehmen des Deckels (11c,11d) zusammen mit dem Shuttle (16) von dem Behälter;
c) Halten des Deckels (11c,11d) und zugleich Anbringen des Shuttles (16) an dem proximalen Körperabschnitt (12);
d) Lösen des Deckels (11c,11d) von dem Shuttle (16); und
e) Anbringen eines distalen Kanülenabschnitts (14) der Intraokularlinse-Inj ektionsvorrichtung (10) an dem proximalen Körperabschnitt (12).

3. Verfahren nach Anspruch 1, ferner mit den folgenden Schritten:
a) Bereitstellen eines proximalen Körperabschnitts (12) der Intraokularlinse-Injektionsvorrichtung (10) und eines gleitbar in dem proximalen Körperabschnitt (12) untergebrachten Kolbens (20);
b) Öffnen des Behälters (11) und Abnehmen des Deckels (11c,11d) zusammen mit dem Shuttle (16) von dem Behälter (11);
c) Halten des Deckels (11c,11d) und zugleich Anbringen des Shuttles (16) an einem distalen Kanülenabschnitt (14) der Intraokularlinse-Injektionsvorrichtung (10);
d) Lösen des Deckels (11c,11d) von dem Shuttle (16); und
e) Anbringen des distalen Kanülenabschnitts (14) mit dem Shuttle (16) an dem proximalen Körperabschnitt (12).

4. Verfahren zum Laden einer Intraokularlinse (30) in einen Abschnitt einer Intraokularlinse-Injektionsvorrichtung (10) mit den folgenden Schritten:
a) Bereitstellen eines distalen Kanülenabschnitts (14) der Intraokularlinse-Injektionsvorrichtung (10);
b) Bereitstellen eines Shuttles (16) und Anordnen der Intraokularlinse (30) darin und Anbringen des Shuttles (16) an dem distalen Kanülenabschnitt (14);
c) Bereitstellen eines Behälters (11) mit einem Deckel (11c,11d);
d) lösbares Verbinden des distalen Kanülenabschnitts (14) mit dem Deckel (11c,11d) und Versiegeln des distalen Kanülenabschnitts (14), des Shuttles (16) und der darin angeordneten Intraokularlinse (30) in dem Behälter (11).

5. Verfahren nach Anspruch 4, ferner mit den folgenden Schritten:
a) Bereitstellen eines proximalen Körperabschnitts (12) der Intraokularlinse-Injektionsvorrichtung (10) und eines gleitbar in dem proximalen Körperabschnitt (12) untergebrachten Kolbens (20);
b) Öffnen des Behälters (11) und Abnehmen des Deckels (11c,11d) zusammen mit dem distalen Kanülenabschnitt (14) und dem Shuttle (16) von dem Behälter (11);
c) Halten des Deckels (11c,11d) und zugleich Anbringen des distalen Kanülenabschnitts (14) an dem proximalen Körperabschnitt (12); und
d) Lösen des Deckels (11c,11d) von dem distalen Kanülenabschnitt (14).

6. Verfahren nach Anspruch 2, 3 oder 5, ferner mit dem Schritt des separaten Ladens des proximalen Körperabschnitts (12) und des distalen Kanülenabschnitts (14) aus dem Behälter (11).

7. Verfahren nach Anspruch 1, ferner mit dem Schritt des Sterilisierens des Shuttles (16) und der Intraokularlinse (30), wenn sie in dem Behälter (11) versiegelt sind.

8. Verfahren nach Anspruch 7, wobei der Sterilisationsschritt eine Dampfsterilisation ist.

9. Verfahren nach Anspruch 1, wobei das Shuttle mittels Schnappverbindung mit dem proximalen Körperabschnitt (12) verbunden wird.

10. Verfahren nach Anspruch 1, wobei der proximale Körperabschnitt (12) und der distale Kanülenabschnitt (14) mittels Schnappverbindung miteinander verbunden werden.

11. Verfahren zur gebrauchsfertigen Vorbereitung einer Injektorvorrichtung mit den folgenden Schritten:
a) Bereitstellen eines proximalen Körperabschnitts (12) der Injektionsvorrichtung mit einem Längskanal;
b) Bereitstellen eines distalen Kanülenabschnitts (14) der Injektionsvorrichtung mit einem Längskanal;
c) Bereitstellen eines Shuttles (16) mit einem Längskanal und einer in dem Shuttle (16) angeordneten Intraokularlinse (30);
d) Bereitstellen eines Behälters (11) mit einem Deckel (11c,11d) zum Versiegeln eines offenen Endes des Behälters (11), wobei der Deckel (11c,11d) lösbar mit dem Shuttle (16) verbunden ist und der Deckel (11c,11d) so angeordnet ist, dass das Shuttle (16) und die Intraokularlinse (30) im Inneren des Behälters (11) versiegelt sind;
e) Herausnehmen des Shuttles (16) aus dem Behälter (11) unter Verwendung des Deckels (11c,11d);
f) Anbringen des Shuttles (16) an dem proximalen Körperabschnitt (12) oder dem distalen Kanülenabschnitt (14) unter Verwendung des Deckels (11c,11d); und
g) Lösen des Behälterdeckels (11c,11d) von dem Shuttle (16).

12. Verfahren nach Anspruch 11, ferner mit Anbringen des distalen Kanülenabschnitts (14) an dem proximalen Körperabschnitt (12).

13. Anordnung zur gebrauchsfertigen Vorbereitung einer Injektorvorrichtung mit:
a) einem proximalen Körperabschnitt (12) der Injektionsvorrichtung (10) mit einem Längskanal;
b) einem distalen Kanülenabschnitt (14) der Injektionsvorrichtung (10) mit einem Längskanal;
c) einem Shuttle (16) mit einem Längskanal und einer in dem Shuttle (16) angeordneten Intraokularlinse (30);
d) einem Behälter (11) mit einem Deckel (11c,11d) zum Versiegeln eines offenen Endes des Behälters (11), wobei der Deckel (11c,11d) lösbar mit dem Shuttle (16) verbunden ist und der Deckel (11c,11d) so angeordnet ist, dass das Shuttle (16) und die Intraokularlinse (30) im Inneren des Behälters (11) versiegelt sind; wobei
e) das Shuttle (16) unter Verwendung des Deckels (11c,11d) aus dem Behälter (11) herausnehmbar ist;
f) das Shuttle (16) unter Verwendung des Deckels (11c,11d) an dem proximalen Körperabschnitt (12) oder dem distalen Kanülenabschnitt (14) anbringbar ist; und
g) der Behälterdeckel (11c,11d) von dem Shuttle (16) lösbar ist.

14. Anordnung nach Anspruch 13, ferner mit einer Einrichtung zum Anbringen des Kanülenabschnitts (14) an dem proximalen Körperabschnitt (12).

15. Anordnung nach Anspruch 13 oder 14, wobei der proximale Körperabschnitt (12) und der distale Kanülenabschnitt (14) koaxial aneinander angebracht sind und wobei der distale Kanülenabschnitt (14) an seinem dem proximalen Körperabschnitt (12) entgegengesetzten Ende eine distale Spitze aufweist, durch welche hindurch die Intraokularlinse (30) ausgestoßen werden kann, ferner mit:
einem Kolben (20) in teleskopischer Beziehung mit dem proximalen Körperabschnitt (12) und dem distalen Kanülenabschnitt (14); und wobei
der Kolben (20) durch den gesamten distalen Kanülenabschnitt (14) vorgeschoben werden kann, wodurch die Intraokularlinse (30) aus der distalen Spitze ausgestoßen wird.

16. Anordnung nach einem der Ansprüche 13 bis 15, wobei das Shuttle (16) mittels Schnappverbindung mit dem proximalen Körperabschnitt (12) verbunden werden kann.

17. Anordnung nach einem der Ansprüche 13 bis 16, wobei der proximale Körperabschnitt (12) und der distale Kanülenabschnitt (14) mittels Schnappverbindung miteinander verbunden werden können.

18. Behälter (11) mit einem Deckel (11c,11d) zum Versiegeln eines offenen Endes des Behälters (11), einem Shuttle (16) mit einem Längskanal und einer in dem Shuttle (16) angeordneten Intraokularlinse (30),
wobei der Deckel (11c,11d) so angeordnet ist, dass das Shuttle (16) und die Intraokularlinse (30) im Inneren des Behälters (11) versiegelt sind; wobei das Shuttle (16) unter Verwendung des Deckels (11c,11d) aus dem Behälter (11) herausnehmbar ist.

19. Behälter nach Anspruch 18, wobei das Shuttle (16) unter Verwendung des Deckels (11c,11d) an einem proximalen Körperabschnitt (12) oder einem distalen Kanülenabschnitt (14) einer Injektorvorrichtung (10) anbringbar ist.

20. Behälter nach Anspruch 19, wobei das Shuttle (16) mittels Schnappverbindung mit dem proximalen Körperabschnitt (12) verbunden werden kann.

## Revendications

1. Procédé pour emballer une lentille intraoculaire (30) dans une partie d'un dispositif d'injection de lentille intraoculaire (10) comprenant une navette (16), dans lequel le procédé comprend les étapes consistant à:
a) prévoir la navette (16) et positionner la lentille intraoculaire (30) à l'intérieur de cette dernière;
b) prévoir un flacon (11) avec un couvercle (11c, 11d);
c) raccorder de manière détachable ladite navette (16) audit couvercle (11c, 11 d) et fermer hermétiquement ladite navette (16) avec ladite lentille intraoculaire (30) à l'intérieur de cette dernière dans ledit flacon (11).

2. Procédé selon la revendication 1, et comprenant en outre les étapes consistant à:
a) prévoir une section de corps proximale (12) du dispositif d'injection de lentille intraoculaire (10) et un piston plongeur (20) reçu de manière coulissante dans ladite section de corps proximale (12);
b) ouvrir le flacon (11) et retirer ledit couvercle (11c, 11d) et la navette (16) ensemble dudit flacon;
c) tout en maintenant ledit couvercle (11c, 11d), fixer la navette (16) sur ladite section de corps proximale (12);
d) retirer ledit couvercle (11c, 11d) de ladite navette (16); et
e) fixer une section de buse distale (14) du dispositif d'injection de lentille intraoculaire (10) à ladite section de corps proximale (12).

3. Procédé selon la revendication 1 et comprenant en outre les étapes consistant à:
a) prévoir une section de corps proximale (12) du dispositif d'injection de lentille intraoculaire (10) et un piston plongeur (20) reçu de manière coulissante dans ladite section de corps proximale (12);
b) ouvrir le flacon (11) et retirer ledit couvercle (11c, 11d) et la navette (16) ensemble dudit flacon (11);
c) tout en maintenant ledit couvercle (11c, 11d), fixer la navette (16) sur une section de buse distale (14) du dispositif d'injection de lentille intraoculaire (10);
d) retirer ledit couvercle (11c, 11d) de ladite navette (16); et
e) fixer ladite section de buse distale (14) avec ladite navette (16) sur ladite section de corps proximale (12).

4. Procédé pour emballer une lentille intraoculaire (30) dans une partie d'un dispositif d'injection de lentille intraoculaire (10) comprenant les étapes consistant à:
a) prévoir une section de buse distale (14) du dispositif d'injection de lentille intraoculaire (10);
b) prévoir une navette (16) et positionner la lentille intraoculaire (30) à l'intérieur de cette dernière et fixer ladite navette (16) sur ladite section de buse distale (14);
c) prévoir un flacon (11) avec un couvercle (11c, 11d);
d) raccorder de manière détachable ladite section de buse distale (14) audit couvercle (11c, 11d) et fermer hermétiquement ladite section de buse distale (14), ladite navette (16) et ladite lentille intraoculaire (30) à l'intérieur dans ledit flacon (11).

5. Procédé selon la revendication 4, et comprenant en outre les étapes consistant à:
a) prévoir une section de corps proximale (12) du dispositif d'injection de lentille intraoculaire (10) et un piston plongeur (20) reçu de manière coulissante dans ladite section de corps proximale (12);
b) ouvrir le flacon (11) et retirer ledit couvercle (11c, 11d), ladite section de buse distale (14) et ladite navette (16) ensemble dudit flacon (11);
c) tout en maintenant ledit couvercle (11e, 11d), fixer ladite section de buse distale (14) sur ladite section de corps proximale (12); et
d) retirer ledit couvercle (11c, 11d) de ladite section de buse distale (14).

6. Procédé selon la revendication 2, 3 ou 5, comprenant en outre l'étape consistant à emballer ladite section de corps proximale (12) et ladite section de buse distale (14) séparément dudit flacon (11).

7. Procédé selon la revendication 1, comprenant en outre l'étape consistant à stériliser ladite navette (16) et ladite lentille intraoculaire (30) lorsqu'elles sont hermétiquement enfermées dans ledit flacon (11).

8. Procédé selon la revendication 7, dans lequel ladite étape de stérilisation est la stérilisation à la vapeur.

9. Procédé selon la revendication 1, dans lequel ladite navette est encliquetée sur ladite section de corps proximale (12).

10. Procédé selon la revendication 1, dans lequel ladite section de corps proximale (12) et ladite section de buse distale (14) sont encliquetées ensemble.

11. Procédé pour préparer un dispositif d'injection destiné à être utilisé, comprenant les étapes consistant à:
a) prévoir une section de corps proximale (12) du dispositif d'injection ayant une voie de passage longitudinale;
b) prévoir une section de buse distale (14) du dispositif d'injection ayant une voie de passage longitudinale;
c) prévoir une navette (16) ayant une voie de passage longitudinale et une lentille intraoculaire (30) positionnée dans ladite navette (16);
d) prévoir un flacon (11) ayant un couvercle (11c, 11 d) pour fermer hermétiquement une extrémité ouverte dudit flacon (11), le couvercle (11c, 11d) étant raccordé de manière détachable à ladite navette (16), ledit couvercle (11c, 11d) étant positionné pour fermer hermétiquement ladite navette (16) et la lentille intraoculaire (30) à l'intérieur dudit flacon (11);
e) retirer ladite navette (16) dudit flacon (11) à l'aide dudit couvercle (11c, 11d);
f) fixer ladite navette (16) sur ladite section de corps proximale (12) ou ladite section de buse distale (14) à l'aide dudit couvercle (11c, 11d); et
g) libérer ledit couvercle de flacon (11c, 11d) de ladite navette (16).

12. Procédé selon la revendication 11, comprenant en outre l'étape consistant à fixer ladite section de buse distale (14) à ladite section de corps proximale (12).

13. Kit pour préparer un dispositif d'injection (10) destiné à être utilisé, comprenant:
a) une section de corps proximale (12) du dispositif d'injection (10) ayant une voie de passage longitudinale;
b) une section de buse distale (14) du dispositif d'injection (10) ayant une voie de passage longitudinale;
c) une navette (16) ayant une voie de passage longitudinale et une lentille intraoculaire (30) positionnée dans ladite navette (16);
d) un flacon (11) ayant un couvercle (11c, 11d) pour fermer hermétiquement une extrémité ouverte dudit flacon (11), le couvercle (11c, 11d) étant raccordé de manière détachable à ladite navette (16), ledit couvercle (11c, 11d) étant positionné pour fermer hermétiquement ladite navette (16) et la lentille oculaire (30) à l'intérieur dudit flacon (11); dans lequel:
e) ladite navette (16) est détachable dudit flacon (11) à l'aide dudit couvercle (11c, 11 d) ;
f) ladite navette (16) peut être fixée sur ladite section de corps proximale (12) ou ladite section de buse distale (14), à l'aide dudit couvercle (11c, 11d); et
g) ledit couvercle (11c, 11d) de flacon est détachable de ladite navette (16).

14. Kit selon la revendication 13, comprenant en outre des moyens pour fixer ladite section de buse (14) à ladite section de corps proximale (12).

15. Kit selon la revendication 13 ou 14, dans lequel ladite section de corps proximale (12), et ladite section de buse distale (14) sont fixées de manière coaxiale et ladite section de buse distale (14) comprend une pointe distale au niveau de son extrémité opposée à ladite section de corps proximale (12), à travers laquelle ladite lentille intraoculaire (30) peut être extraite, comprenant en outre:
un piston plongeur (20) en relation télescopique à l'intérieur desdites sections de corps proximale (12) et de buse distale (14); et dans lequel:
ledit piston plongeur (20) est adapté pour être entièrement avancé à travers ladite section de buse distale (14) extrayant ainsi ladite lentille intraoculaire (30) par ladite pointe distale.

16. Kit selon l'une quelconque des revendications 13 à 15, dans lequel ladite navette (16) est adaptée pour être encliquetée sur ladite section de corps proximale (12).

17. Kit selon l'une quelconque des revendications 13 à 16, dans lequel ladite section de corps proximale (12) et ladite section de buse distale (14) sont adaptées pour être encliquetées ensemble.

18. Flacon (11) ayant un couvercle (11c, 11d) pour fermer hermétiquement une extrémité ouverte dudit flacon (11) et une navette (16) ayant une voie de passage longitudinale et une lentille intraoculaire (30) positionnée dans ladite navette (16),
ledit couvercle (11c, 11d) étant positionné pour fermer hermétiquement ladite navette (16) et ladite lentille intraoculaire (30) à l'intérieur dudit flacon (11); dans lequel ladite navette (16) est détachable dudit flacon (11) à l'aide dudit couvercle (11c, 11d).

19. Flacon selon la revendication 18, dans lequel ladite navette (16) peut être fixée sur une section de corps proximale (12) ou une section de buse distale (14) d'un dispositif d'injection (10) à l'aide dudit couvercle (11c, 11d).

20. Flacon selon la revendication 19, dans lequel ladite navette (16) est adaptée pour être encliquetée sur ladite section de corps proximale (12).
